# EUROPEAN PATENT SPECIFICATION

(11) **EP 0 045 194 B1**
(45) Date of publication and mention of the grant of the patent: **20.03.1985**
(21) Application number: 81303405.5
(22) Date of filing: 24.07.1981
(51) Int. Cl.: A61K 7/16, C08L 81/06, C08G 75/20

(54) **Oral hygiene compositions comprising Sulfonated poly (arylene ether sulfone) polymers as dental plaque barriers**
Oralhygienische Sulfonierte Poly(arylen-äther-sulfon)polymere als Schutzfilm gegen Zahnbelagenthaltende Zusammensetzungen
Compositions pour l'hygiène orale comprenant des polymères de poly (arylène éther sulfones) sulfonés comme barrière contre la plaque dentaire

(30) Priority: 25.07.1980 US 172487; 25.07.1980 US 172347; 25.07.1980 US 172350
(43) Date of publication of application: 03.02.1982
(73) Proprietor: Johnson & Johnson Products Inc., New Brunswick New Jersey 08903 (US)
(72) Inventor: Buck, Carl J., Berkeley Heights New Jersey 07922 (US); Sipos, Tibor, Lebanon New Jersey (US); Vidra, James D., Clinton New Jersey (US)
(74) Representative: Jones, Alan John

## Description

### Technical Field

This invention relates to oral hygiene compositions for preventing attachment of bacteria .to teeth. More particularly, it relates to compositions comprising certain sulfonated polymeric materials that have been found useful in inhibiting the agglutination of oral microbes on teeth.

### Background Art .

The prevention of the deposition of dental plaque on teeth is a highly desired result. Dental plaque results when cariogenic bacteria aggregate in colonies on the surface of teeth and form a tenacious deposit thereon. The presence of plaque on teeth is believed to be a precursor to development of gingivitis, dental caries and periodontal disease.

While many attempts have been made to control the effects of cariogenic bacteria and the dental plaque they produce, for example, fluoride, flossing, brushing, etc., treatments, these are typically directed to either counteracting the secondary effects of plaque on the teeth and gums, or to the removal of plaque that is already formed on and adhering to the teeth and surrounding tissue. Such treatments are not, however, entirely successful, and must be supplemented with periodic treatment by dental professionals. To date, there is no commercially feasible home treatment method for preventing the formation of plaque or its adhesion to teeth.

### The Invention

According to the present invention, there is provided an oral hygiene composition comprising an effective amount for preventing attachment of dental plaque to teeth of a sulfonated poly(arylene ether sulfone) polymer having repeating units selected from the group consisting of structure (A). 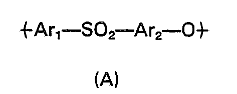 and structure (B), 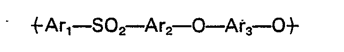 wherein Ar₁ and Ar₂ are each selected from 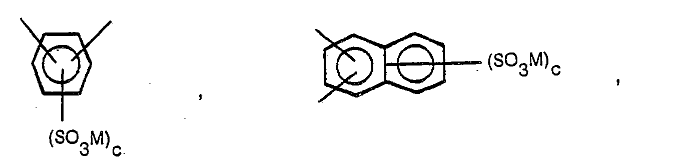 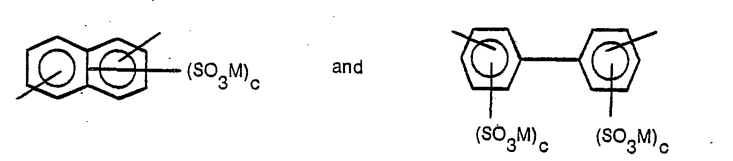 provided further that Ar₂ also can comprise one or more spacing units selected from -Ar4-SOZ-Ar4-and Ar₄―SO₂―Ar₄―SO₂―Ar₄― , each Ar₄ in said spacing units being separately selected from 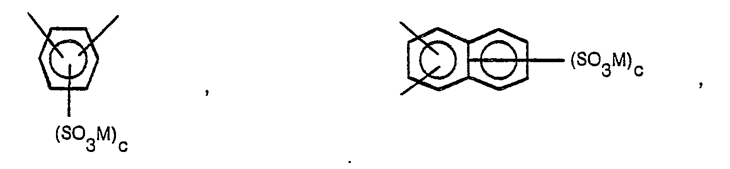 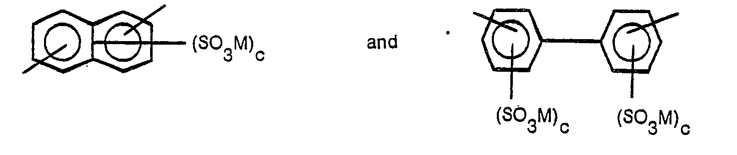 Ar₃ is selected from Ar4 and 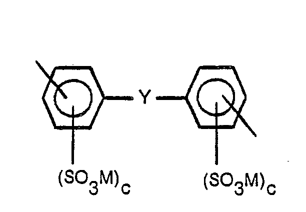 wherein Y is selected from lower alkylene having 1-5 carbon atoms, lower alkylidene having 2-5 carbon atoms, 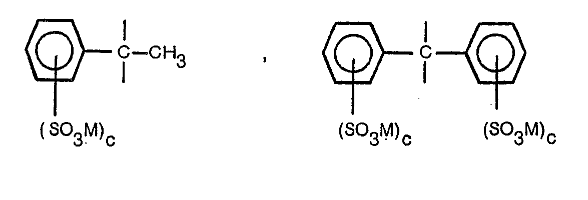 0, S, and S0₂; subscript c being an integer selected from 0, 1, and 2, the average quotient obtained by dividing the sum of the c's within each of repeating units (A) and (B) by the number of aromatic groups in said repeating unit being at least 0.2; and M is selected from lithium, sodium, potassium, calcium, magnesium, zinc, aluminium, hydrogen, ammonium, and substituted ammonium ions derived from pharmaceutically acceptable organic amines, in a pharmaceutically acceptable oral hygiene vehicle compatible with said polymer.

IN the formulae given above and in the remainder of the description and the claims, a line connecting substituent groups to the 9, 10 bond in the naphthylene groups indicates that the or each substituent group can be attached to any free carbon atom of the naphthylene group.

The hydrophilic sulfonic acid and sulfonic acid salt derivatives of certain poly (arylene ether sulfone) polymers referred to above have been synthesized and found to inhibit the deposition of dental plaque onto human teeth. These hydrophilic polymeric sulfonates have good film forming characteristics and, accordingly, are applied to teeth from various dentifrice formulations, mouth rinses, or other oral hygiene compositions. The sulfonate polymers are anionic in nature and susbtantially soluble in water or water/ organic solvent vehicles, primarily because of the relatively high degree of sulfonation achieved during preparation of these derivatives. While the mechanism of action of the hydrophilic polymeric films in retarding plaque deposition is not known with absolute certainty, it is presumed that the films of anionically-charged polymers deposited on teeth effect a mutual repulsion between the negatively charged polymer film and the negatively charged microorganisms in oral fluids responsible for plaque generation. For example, when powdered human dental enamel is dispersed in the aqueous media containing salts of the polymeric sulfonates, a substantially negative surface charge is imparted to the enamel particles, as determined by zeta potential measurements. The compositions, comprising the sulfonated poly (arylene ether sulfone) polymers, of this invention are especially effective as dentifrices and other oral hygiene preparations in reducing dental plaque deposition on teeth.

In accordance with a preferred embodiment of the present invention, it has now been found that the zinc salts of the foregoing sulfonated poly(arylene ether sulfone) polymers exhibit surprisingly superior substantivity to human dental enamel, as determined, e.g., by zeta potential measurements, after repeated washings with water to that exhibited by other salts. Accordingly, the present invention also provides highly substantive oral hygiene compositions for reducing dental plaque deposition on teeth comprising a zinc salt of a sulfonated poly(arylene ether sulfone) polymer, as more fully defined above, in a pharmaceutically acceptable oral hygiene delivery system compatible therewith.

In yet another preferred embodiment, the invention provides an oral hygiene composition for inhibiting the attachment of bacterial plaque to teeth, which comprises water, at least 5% by weight of a humectant selected from the group consisting of glycerol and sorbitol, at least 16% by weight of a gelling agent selected from the group consisting of the block copolymers of polyoxypropylene and polyoxyethylene wherein the polyoxypropylene component has a molecular weight in the range of from 3,000 to 4,000 and the polyoxyethylene comprises between 25 and 80 mole % of said copolymer, and from 0.5 to 10% by weight of the plaque barrier agent, which is a sulfonated poly(arylene ether sulfone) polymer as described above. Thus, only these gels, of all dentifrice vehicle systems tested, have the combination of high plaque barrier activity, high substantivity and good aesthetic qualities. Desirable aesthetic properties for the gels of this invention can be defined as clarity, lack of cloudiness, a translucent gel possessing a firmness that prevents absorption or dissolution of the gel into toothbrush bristles. By contrast, poor aesthetics includes gel separation, precipitation of active ingredient and cloudiness of gel.

The plaque barrier agents are present in these formulations in effective concentrations in the range of from 0.5 to 10 weight percent. A more preferred range is from 2 to 8 percent by weight, 5% being the presently most preferred concentration.

Examples of specific, commercially available polyoxypropylene/polyoxyethylene block copolymer gelling agents within the foregoing definition are Pluronic® F―127, Pluronic® F―108, Pluronic@ F―103, Pluronic® P―104, Pluronic® P―105, and Pluronic® P―123. Effective gelling agent concentrations are generally between 16% and 22% by weight.

Among the advantages of the preferred nonabrasive gel formulations of the present invention are the high level of plaque barrier activity and substantivity that are maintained in a clear, uniform, aesthetically pleasing preparation that is acceptable for use as an oral hygiene product. Moreover, since such anticaries fluoride compounds as sodium fluoride can be incorporated in these compositions, they can be employed as the primary dentifrice in an oral hygiene prophylaxis program.

Hydrophilic polymeric, anionic sulfonates useful for dental plaque control in accordance with the present invention are prepared by aromatic sulfonation of poly(arylene ether sulfone) polymers, followed by conversion of the polymeric sulfonic acid derivatives to metal salts of certain of the Group IA alkali metals, Groups IIA, IIB, and IIIA multi-valent metals, and ammonium or amine-salts.

The repeating units of the sulfonated poly(arylene ether sulfone) polymers of this invention are selected from the group consisting of structure (A), 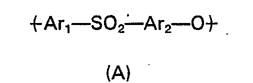 and structure (B), 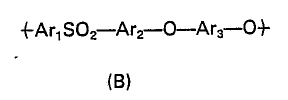 wherein Ar₁ and Ar₂ are each selected from 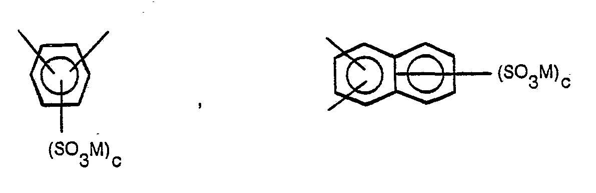 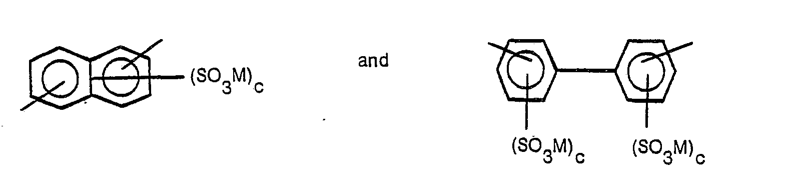 provided further that Ar₂ also can comprise one or more spacing units selected from -Ar₄-S0₂-Ar₄-and -Ar4-S02-Ar4-S02-Ar4-, each Ar4 in said spacing units being separately selected from 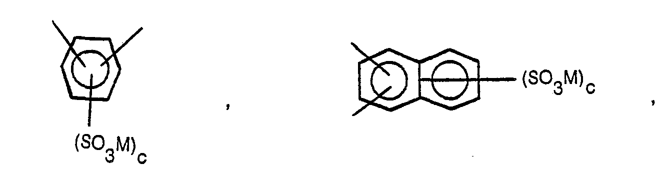 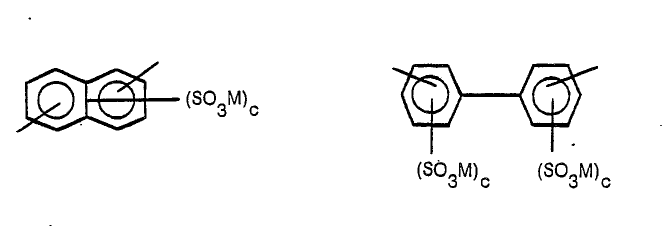 Ar₃ is selected from Ar4 and 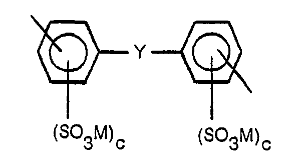 where Y is selected from lower alkylene having 1-5 carbon atoms, lower alkylidine having 2-5 carbon atoms, 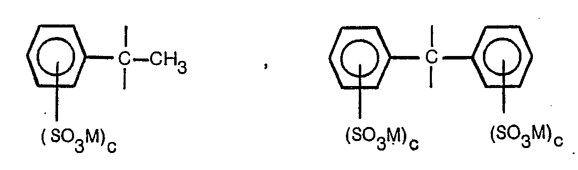 O, S, and SO₂; subscript c being an integer selected from 0, 1 and 2; the quotient, obtained by dividing the total number of sulfonate groups (i.e. the sum of the c's within each of repeating units (A) and (B) by the number of aromatic groups in said repeating unit, being (on the average) at least 0.2; and M is selected from the group consisting of lithium, sodium, potassium, calcium, magnesium, zinc, aluminium, hydrogen, ammonium, and substituted ammonium ions derived from pharmaceutically acceptable organic amines.

In general, the metal and ammonium salts are preferred over the free sulfonic acid forms of the polymers because of their higher water solubility and lower degree of acidity, thereby favoring their use in oral hygiene formulations as dental plaque control barriers. The zinc salts are particularly preferred.

The polymers utilized for conversion to sulfonate derivatives are available either commercially or synthesized by known procedures found in the literature. Representative examples of commercial poly (arylene ether sulfone) polymers which can be sulfonated to the hydrophilic, anionic sulfonates of this invention are the following:
(a) Udel@ Polysulfone, type P1700 or medical grade MG11, available from Union Carbide Corp. in a molecular weight of about 35,000, and having the following repeating unit structure: 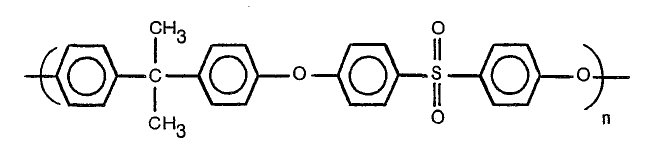
(b) Victrex@ Polyethersulfone, grades 100P, 200P, 300P from ICI America, Inc.: 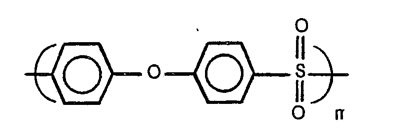
(c) Radel® Polysulfone from Union Carbide Corp., and thought to have the following repeating unit structure:

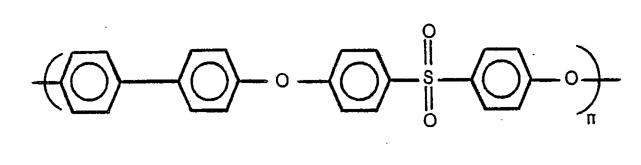

The generalized structures for other poly (arylene ether sulfone) polymers that can be sulfonated to form the sulfonated polymers of this invention are represented as formulas (I) and (II), and their method of synthesis is indicated in equations (1) and (2) below: 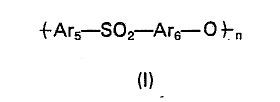 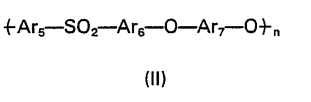 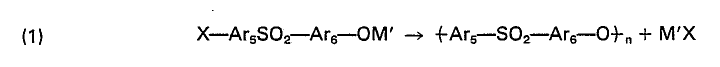 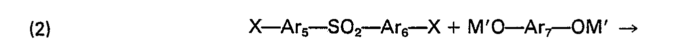 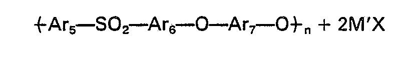 wherein X is a halogen; M' is a univalent metal such as sodium or potassium;
Ar₅ and Ar₆ are each selected from 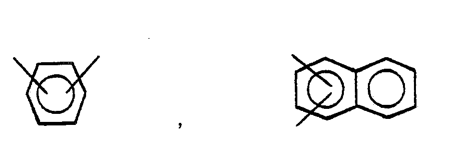 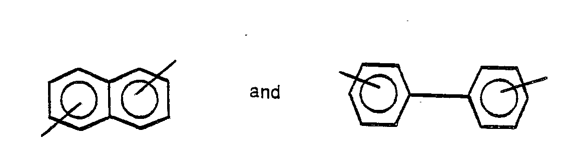 provided further that Are also can comprise one or more spacing units selected from ―Ar₈―SO₂―Ar₈― and ―Ar₈SO₂―Ar₈―SO₂―Ar₈―, each Ar₈ in said spacing units being separately selected from 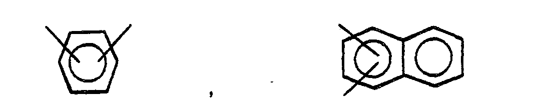 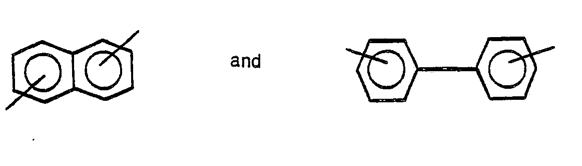 Ar₇ is selected from Ar₈ and 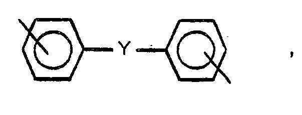 where Y is selected from lower alkylene having 1-5 carbon atoms, lower alkylidene having 2-5 carbon atoms, 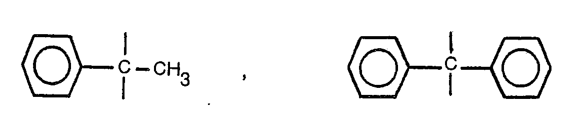 O, S, and SO₂,

Polymers of structure (I) can be synthesized by the general procedure (summarized in equation (1) above) described by T. E. Attwood, et al., in Polymer, Volume 18, pages 354-374 (1977). Polymers of structure (II) are prepared by reaction of bis(haloaryl) sulfones with univalent metal salts of aromatic diols, such as the reaction taught by R. N. Johnson, et. al., J. Polymer Science, Part A-1, Volume 5, pp 2375-2398 (1967). Poly (arylene ether sulfone) polymers suitable for conversion to sulfonated derivatives for use in the compositions of the present invention can be synthesized by varying the nature of the aromatic group, orientation of the linkages on the aromatic ring, and spacing of the sulfone (S0₂), ether (0) and other connecting groups in accordance with the foregoing definitions of the aromatic polymeric structures (I) and (II).

The sulfonation of poly (arylene ether sulfohe) polymers, such as Udel@ Polysulfone, to water insoluble sulfonated polymers with low degrees of sulfonation, and suitable as membranes for water desalination, have been described in the literature and patent publications such as A. Noshay and L. M. Robeson, J. Applied Polymer Science, 20, 1885-1903 (1976); C. L. Brousse, et. al., Desalination, 18, 137-153 (1976); and U.S. Patents Nos. 3,709,841 (issued January 9, 1973), 3,855,122 (issued December 17, 1974), and 3,875,096 (issued April 1, 1975). These sulfonated polysulfones differ from the derivatives of the present invention in that they are substantially water insoluble, due to the relatively low degree of sulfonation, and therefore cannot be utilized in the aqueous media required for oral hygiene applications. As will be described hereinafter, the poly(arylene ether sulfone) sulfonates of the present invention are substantially soluble in water or mixed solvents comprising water and an organic solvent miscible therewith (generally at least 1% w/w) and hydrophilic as a consequence of their higher degree of sulfonation. As discussed in greater detail hereinafter the degree of sulfonation (D.S.) also has a significant effect on the extent of dental plaque deposition. D.S. as used herein is the average number of sulfonate or sulfonic acid groups per repeating unit of the polymeric structure.

Preferred sulfonation agents for preparing the sulfonated polymeric barriers to be used in the compositions of the invention are anhydrous sulfur trioxide, triethyl phosphate (TEP) complexes of sulfur trioxide, and chlorosulfonic acid. Due to the high reactivity of sulfur trioxide and its potent dehydration properties, sulfonation reactions with sulfur trioxide sometimes result in formation of highly insoluble polymer dispersions due to crosslinking caused by inter-polymer chain sulfone formation. In these situations, it is preferable to moderate the sulfonation reactivity by utilization of the sulfur trioxide complexes with triethyl phosphate (TEP), which minimize or essentially eliminate formation of crosslinked by-products [cf. A. F. Turbak, lnd. Eng. Chem., Prod. R & D, 1, 275 (1962); U.S. 3,072,619 (January 8, 1963); A. F. Turbak and A. Noshay, U.S. 3,206,492 (September 14,1965); N. H. Canter, U.S. 3,642,728 (February 15, 1972); A. Noshay and L. M. Robeson, J. Applied Polymer Science, 20,1885 (1976)]. While the sulfonation activity increases with the molar proportion of sulfur trioxide in the complex with TEP, mole ratios of 2:1, 3:1, and 4:1 are preferred for the synthesis of the poly (arylene ether sulfone) sulfonates of the present invention. In some instances where it is difficult to effect sulfonation under milder conditions with the complexes, sulfonation with sulfur trioxide (alone) or chlorosulfonic acid is more effective.

Sulfonations can be effected in solvents such as methylene chloride, 1,2-dichloroethane, and chloroform, since these are generally good solvents for the starting aromatic polymer and poor solvents for the sulfonated polymer, which precipitates directly from the reaction medium and is filtered. In those instances where the product is soluble in the reaction medium and did not precipitate, the sulfonated polymer is isolated by removing the solvent and converted to well-defined solids by either trituration or slurrying with an appropriate non-solvent.

Three modes of reacting the sulfonation agent and polymer were examined: (1) addition of sulfonation agent to polymer, (2) addition of polymer to the sulfonation agent, and (3) simultaneous addition of the sulfonation agent and polymer to the reaction medium. Methods (1) and (3) are preferred, since addition of the polymer to the sulfonation agent (method 2) sometimes gives rise to non-uniform products, probably because of the large excess of sulfonation agent present during the early stages of the reaction. The most preferred sulfonation process is that of method (3), involving simultaneous additions of the reactants. These conditions afford sulfonated products of greater uniformity and which often precipitate directly from the reaction as finely divided solids, thereby minimizing occlusion of solvent, residual acids, complexing agents (e.g., triethyl phosphate), and unreacted polymer by the sulfonated polymer.

Temperature control of the sulfonation reaction with sulfur trioxide and its complexes with TEP is not very critical. Acceptable results are obtained over a -20°C to +40°C range. Sulfonations are generally effected at ambient room temperatures, since the sulfonation exotherm is very mild and rarely results in temperature increases beyond 35°C.

Typical impurities in the sulfonated polymer are small amounts of unreacted polymer, excess sulfonation agent (as sulfuric acid), and residual triethyl phosphate which are occluded in the solid polymer. Substantial purification is effected by slurrying the polymeric sulfonic acid derivatives in nonsolvents therefor, such as the halocarbons.

Removal of the free sulfuric acid is difficult, since it complexes strongly with the polymeric product. It has been found that diethyl ether is an exceptionally good complexing agent for sulfuric acid and effectively removes this contaminant when freshly isolated polymeric solids are slurried in the ether and filtered. Other effective additives for sulfuric acid removal are halocarbon solvent blends with diethyl ether and other oxygenated solvents, such as ethyl acetate and acetone. The sulfuric acid, if not removed, results in contamination of the metal salts prepared by neutralization or ion-exchange reactions on the polymeric sulfonic acid intermediates, with considerable inorganic sulfate, such as sodium sulfate, in the case where the sodium sulfonate polymer is produced.

Efficient purificaton of the sulfonic acid derivatives of the polymers is not always possible, but it has been found that additional purification results in conversion of the sulfonic acid groups to their various salts of monovalent and divalent metal atoms. For example, neutralization of an ethanol solution of Udel@ polysulfone sulfonic acid of D.S. 1.8 with alcoholic sodium hydroxide results in precipitation of the sodium sulfonate salt in a higher state of purity. Much of the occluded triethyl phosphate and any processing solvents are freed to the filtrate during precipitation of the product.

The preferred process for purification of the sulfonated polymers (both free acids and salts), particularly highly water soluble types, is by dialysis of their aqueous solutions in membrane tubes or hollow fiber dialyzing units having a molecular weight cut-off well below the molecular weight of the polymer. Dialysis removes all of the low molecular weight impurities, triethyl phosphate, and inorganic salts. High purity polymers are isolated as solids by freeze-drying or spray drying the dialyzed polymer solution.

Examples of acceptable metal salts of the polymeric sulfonic acid derivatives of poly (arylene ether sulfone) polymers for use in accordance with this invention are the potassium, lithium, sodium, calcium, magnesium, zinc, and aluminium salts. The zinc salts are particularly preferred, since they exhibit higher substantivity to human dental enamel (after repeated washings with water) than the alkali metal salts. Other acceptable salt forms of the polymers are the ammonium salts prepared from ammonia or pharmaceutically-acceptable organic amines.

The alkali metal salts of the sulfonated polymers are conveniently prepared by neutralization of a water or alcohol solution of the polymeric sulfonic acid derivative with alkali metal hydroxide solutions to the potentiometric endpoint. The salts are recovered by filtration, solvent stripping, or freeze drying, depending on the type of solvent used and whether the salt precipitates directly from the solvent medium. Alternatively, sulfonate salts can be prepared by addition of at least stoichiometric quantities of an alkali metal oxide, carbonate, acetate, chloride, nitrate, or sulfate to the sulfonic acid derivative. The salts either precipitate directly, or are isolated by solvent stripping. Purification of the sulfonate salt by dialysis is the preferred procedure for the more highly water soluble salts.

Multivalent metal salts, such as the calcium, magnesium, zinc, and aluminium salts, of the sulfonated polymers can be prepared by methods similar to those described above. In an alternate procedure, multi- valent metal salts can be prepared by an ion-exchange reaction between the multivalent ion and either the free sulfonic acid or an alkali metal sulfonate derivative of the polymer. The neutralization and other salt forming reactions described above are essentially ion-exchange reactions, as typified by the following equations, where P represents the polymer chain:

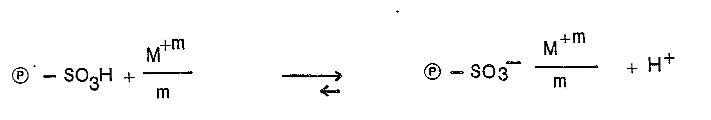

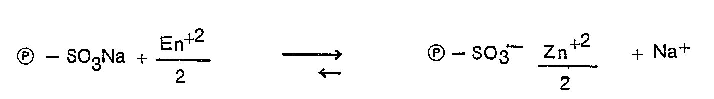

Ammonium salts of the sulfonic acid polymer can be prepared by direct addition of ammonia or a primary, secondary, or tertiary organic amine.

The polymeric sulfonic acids are highly effective in reducing the deposition of plaque during in vitro testing, but these sulfonic acid polymers are too highly acidic to permit use in the oral environment unless suitably buffered. Various salts of the polymeric sulfonic acids are preferred because of their increased solubility in aqueous media and lower degree of acidity. These salts exhibit approximately equivalent reduction of plaque deposition to that exhibited by the corresponding free acids when tested in vitro.

The in vitro test procedure we have employed begins with growth of plaque in small jars containing sterilized trypticase media that has been supplemented with sucrose. Typically, ten jars are individually inoculated with 0.5 ml of unpooled freshly collected human plaque from 10 subjects. In a control series, a presterilized glass slide or an extracted human tooth is inserted into each jar. In the test series, the tooth or glass slide is pretreated with a 1% solution of the test compound (dissolved in water or other vehicle), allowed to dry in order to deposit a thin film of the compound on the surface, and the glass slide or tooth placed in the growth media. The jars are incubated under anaerobic conditions for two days at 37°C. The tooth or glass slide is removed, air dried, and stained with 0.15% FD&C #3 red dye solution to reveal the accumulated plaque deposits. The tooth or glass slide is scored for plaque density on a 0 to 5 scale against the control. Plaque barrier activity is reported as the % of average plaque reduction, as compared to appropriate controls for ten subjects.

A test method used to evaluate substantivity, which is relatively simple to perform and suited to a multicomponent gel formulation in accordance with the present invention, involves variations on the foregoing plaque barrier activity test and is summarized below:

### Test A

1. Coat both sides of a clean glass slide with the test formula. Use a toothbrush to achieve a thin coating.
2. Place the coated glass slide into a jar of preinoculated plaque media.
3. Anaerobically incubate for 48 to 72 hours at 37°C.
4. Stain the plaque deposit on the glass slide by immersing it in a 0.15% solution of FD&C Red #3 for one minute.
5. Evaluate the plaque deposit on the glass slides, comparing the amount of deposit on untreated control slides to the amount of deposit on the treated slides. The difference is reported as the "PERCENT INHIBITION OF PLAQUE ATTACHMENT TO GLASS SLIDES".

### Test B

Follow the method of Test A and make this modification - after coating the slide with the test formula, rinse with cool running (1.6 I/min) tap water for 5 seconds (2.5 seconds/side).

### Test C

Modify the previous test methods by rinsing the glass slide for 10 seconds (5 seconds/side).

### Test D

Modify the test method by rinsing the glass slide for 20 seconds (10 seconds/side).

### Test E

Modify the method of Test A in the following manner:
1. Coat both sides of a clean glass slide with the test formula. Use a toothbrush to achieve a thin coating.
2. Rinse the glass slide with cool running tap water (brushing while rinsing) until all the test formula is washed off (visual observation).
3. Place the glass slide into a jar of pre-inoculated plaque media.
4. Continue as in Test A.

A preferred in vitro test procedure utilized to establish the degree of substantitivy of the sulfonated poly(arylene ether sulfones), but difficult to employ with multi-component gel formulations such as the preferred gel formulations of the present invention, is based on measurement of the zeta potential of powdered human dental enamel which has been contacted with an aqueous solution of the polymeric zinc sulfonate compound. This microelectrophoresis technique involves measurement of adsorption isotherms by determining the zeta potential of dental enamel in the presence of incressing concentrations of the polymeric zinc sulfonate, all solutions being made in 0.0200 M sodium chloride. When Udel@ Polysulfone zinc zulfonate of D.S. 1.8 was so tested, the resultant adsorption isotherms indicated that the surface potential of the tooth enamel particles became increasingly negative with increasing concentration of the zinc salt. A plateau value of about -40mV was reached and indicated that the surface of the enamel was saturated with the polymeric anion. In the absence of any of the polyeric zinc salt, the zeta potential of dental enamel was about -10mV. These experimental techniques established that the polymeric anion is indeed adsorbed on the enamel surface. In substantivity of the polymeric sulfonate, powdered enamel was contacted for a short time with a 0.1% weight/volume solution of the polymeric sulfonate and then, after measurement of the initial charge on the enamel particle, was washed successively with large volumes of 0.0200 M sodium chloride and the zeta potential measured after each wash.

The degree of sulfonation of the poly (arylene ether sulfone) polymer has a significant effect on the reduction of plaque deposition, and it is found that a certain minimal D.S. is required for development of adequate plaque barrier activity. The D.S. can be varied at will by adjusting the conditions of the sulfonation'reaction, such as the molar ratio of sulfonating agent to polymer. The nature of the aromatic polymer repeating unit governs the maximum D.S. which can be achieved. Linking groups, such as ether, sulfone, and various organic radicals (see e.g. the definition of Y set forth above) attached to the aromatic rings in the polymer chain structure can have either a deactivating or activating effect on aromatic sulfonation. Electronic and steric effects determine the position of sulfonation as well as ease of sulfonation. These mechanistic considerations have been reviewed in general organics texts, such as that by R. T. Morrison and R. N. Boyd, "Organic Chemistry", Third Edition, Allyn and Bacon, Inc., Boston, 1973. In the poly (arylene ether sulfone) polymers, the ester linkages activate sulfonation in the available ortho- positions of the adjoining aromatic rings; in contrast, the sulfone group will deactivate the aromatic rings to which it is bonded with respect to aromatic sulfonation. A. Noshay and L. M. Robeson (supra) have established, for example, that sulfonation of Udel@ Polysulfone indeed takes place only in the ortho- positions of the Bisphenol A moiety relative to the ether oxygen atoms, e.g.,

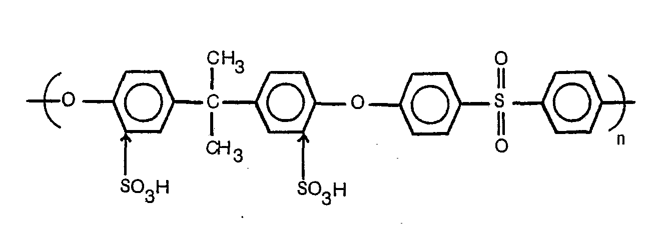

The degree of sulfonation (D.S.) of the poly(arylene ether sulfone) derivative can be determined by any of several methods: (a) NMR analysis (b) elemental analysis for sulfur to carbon ratio, or (c) direct titration of the sulfonic acid with standard sodium hydroxide. The NMR method is perhaps the more exact procedure, since it is not prone to interference by other impurities, such as with the acidimetric or elemental analyses. Acidimetric assays for D.S. agreed well with those determined via NMR when the sulfonic acid polymer is carefuly washed free of entrapped sulfuric acid and thoroughly dried and, in this regard, is often the most convenient assay method for monitoring the progress of the sulfonation reaction. Good correlation between calculated and theoretical values for the metal salt content, determined by atomic absorption, is obtained on polymers carefully purified by dialysis.

The acidimetric procedure for D.S. determination involves titration of an accurately weighed two gram sample (±0.1 mg) of the sulfonic acid polymer, dissolved in about ten volumes of water, alcohol, or other solvents, with standardized sodium hydroxide to the potentiometric endpoint. The acidity, A, of the samples is expressed in milliequivalents/gram (meq/g). Using the acidity value, A, and the formula weight, R, of the unsulfonated repeat unit in the polymer, the D.S. is calculated from the following equations: 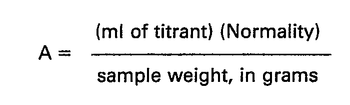 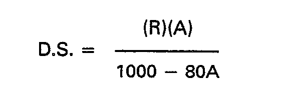 A related concept to D.S. which is sometimes more useful in correlating polymer structure with plaque barrier activity is the average number of sulfonate or sulfonic acid groups per aromatic group in the repeating unit. This is simply the D.S. (as determined by the aforementioned procedures) divided by the number of aromatic groups in the repeating unit, i.e., D.S./Ar. For example, Udel@ Polysulfone sodium sulfonate of D.S. 2.0 can be expressed as exhibiting a D.S./Ar of 0.5, since there are four aromatic groups within each repeating unit.

The plaque barrier activity of the sulfonated salts of the poly (arylene ether sulfone) polymers, such as those of Udel@ Polysulfone, Vitrex@ Polyethersulfone, and Radel@ Polysulfone are shown in Table 1 and demonstrate the necessity of achieving a certain minimum extent of sulfonation in order to obtain satisfactory plaque barrier activity. Generally, sulfonated poly (arylene ether sulfone) polymers of high plaque barrier activity are obtained only when the average number of sulfonate groups per aromatic group (D.S./AR) within the polymer is at least 0.2. Aside from being insoluble in water, the non-sulfonated polymeric intermediates exhibit no plaque barrier properties whatsoever. Effective plaque barrier activity (plaque reduction of at least above 40%) is seen only when the hydrophilic properties of the polymer are increased by introduction of either sulfonic acid or sulfonate salt functional groups.

While the molecular weight of the polymers used in the compositions of th present invention is not considered to be a critical factor, they generally have a weight average molecular weight within the broad range of from 5,000 to 200,000. A preferred molecular weight range is from 20,000 to 50,000.

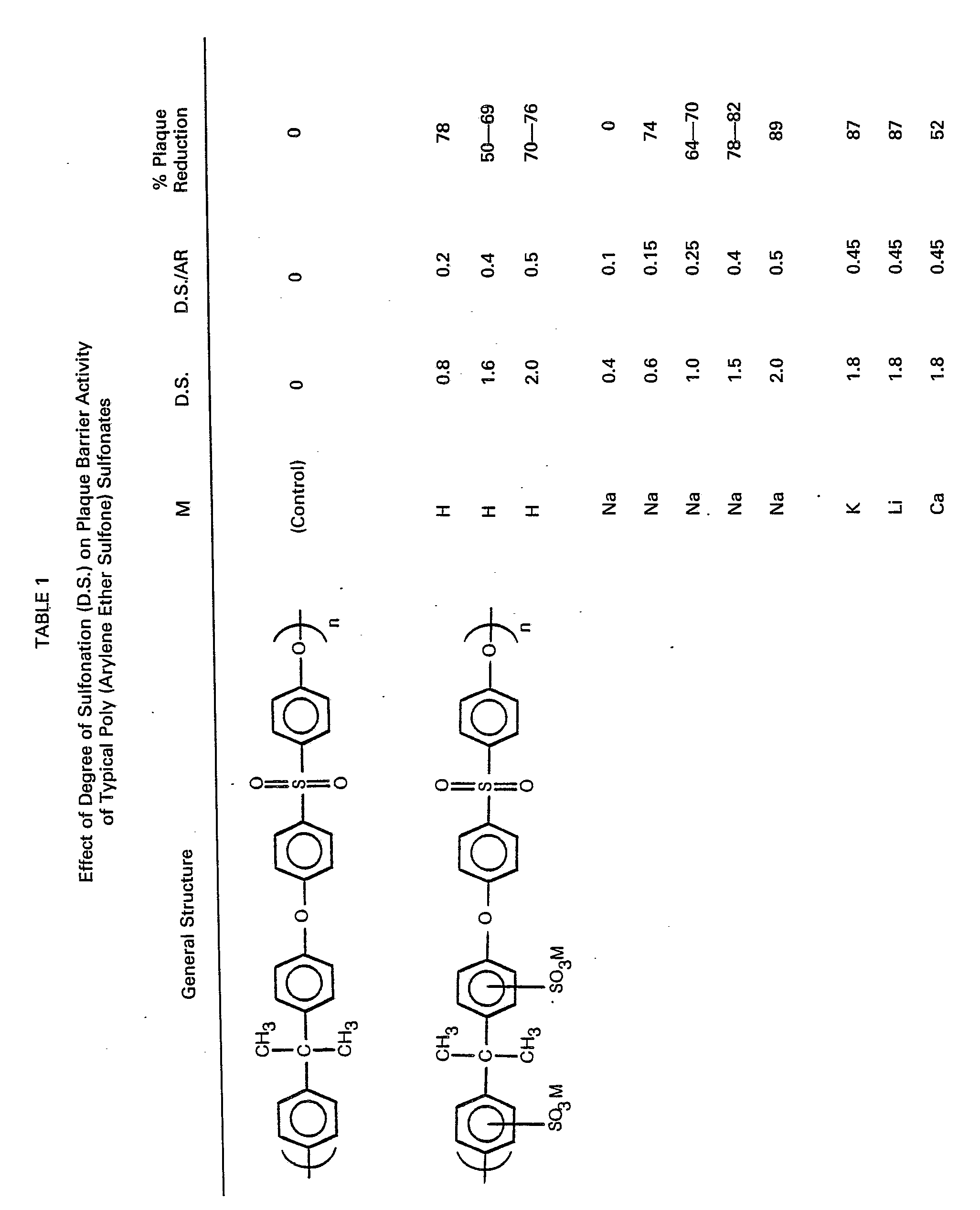

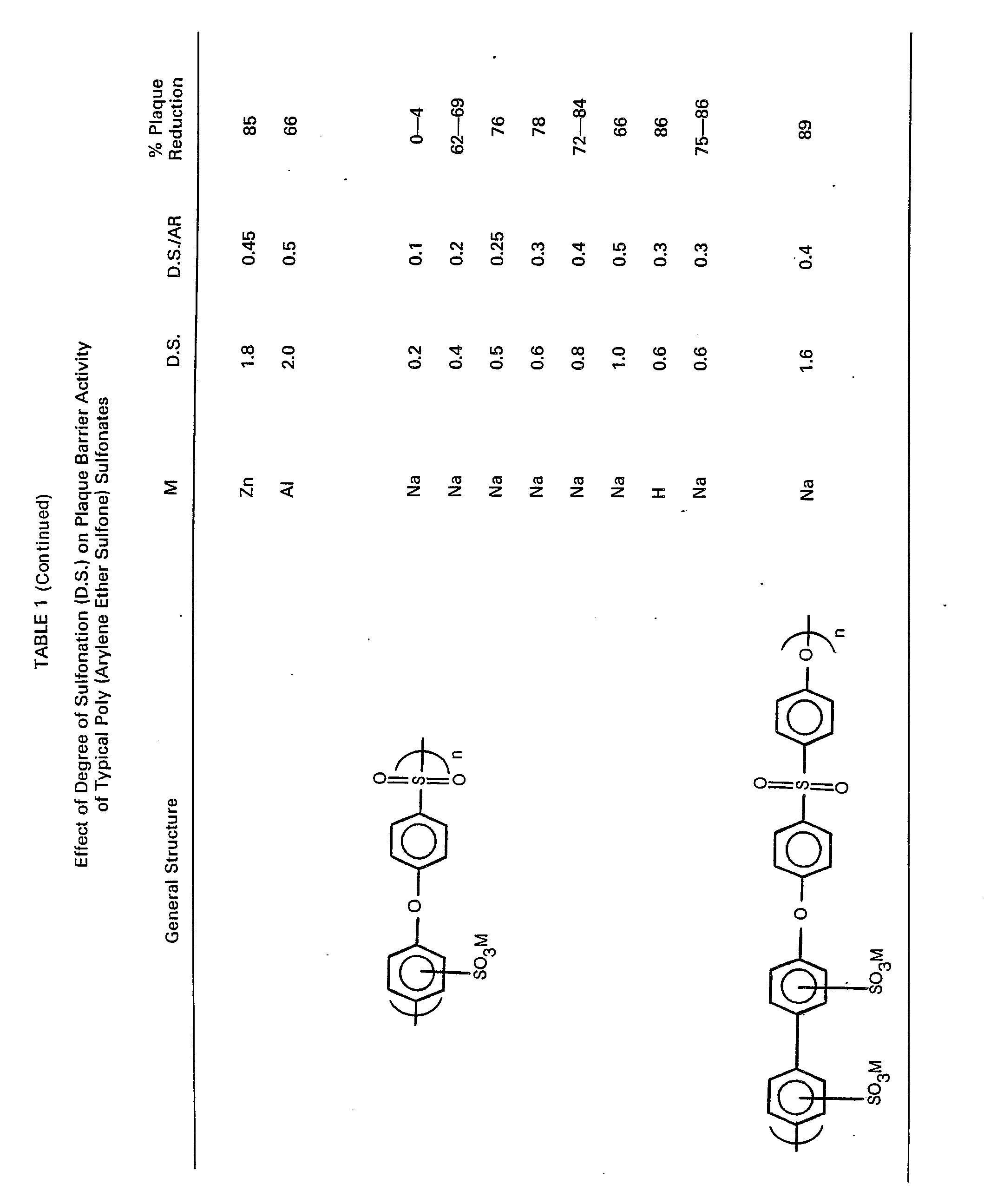

### Example 1

### Udel® Polysulfone Sulfonic Acid, D.S. 1.8

A 12 liter resin flask was fitted with a mechanical stirrer, thermometer, two addition funnels, and a nitrogen inlet adapter. The flask was charged with 3000 ml methylene chloride which was dried over molecular sieves. Into one of the addition funnels was charged a solution of 664 g (1.50 moles) Udel@ Polysulfone (type P1700, medical grade, MG 11; Union Carbide) in 3000 ml dry methylene chloride. Into the other addition funnel was charged the sulfonation agent, prepared by controlled addition of 360 g (4.50 moles) anhydrous liquid sulfur trioxide to a cooled solution of 205 g (1.125 moles) triethyl phosphate dissolved in 3000 ml dry methylene chloride.

While stirring the methylene chloride solvent in the resin flask, the solutions of the polymer and sulfonation agent were added simultaneously over one to two hours at the ambient temperature, varying from 23 to 32°C. After the additions were completed, the resultant suspension of white solids was stirred another one to two hours at the ambient temperature. The product was vacuum-filtered on a glass-fritted funnel, washed three times with 4 liters of methylene chloride by mechanical slurry and filtered each time. A final slurry wash in anhydrous diethyl ether whitened the product. After air drying at room temperature the yield of the sulfonic acid derivative of Udel@ Polysulfone was 1043 grams. The degree of sulfonation (D.S.), determined by acidimetric titration or NMR analysis, was 1.8.

### Example 2

### Udel® Polysulfone Sodium Sulfonate, D.S. 1.8

A stirred solution of 1030 grams of the sulfonic acid derivative, prepared according to Example 1, in 5150 ml 95% ethanol, was stirred vigorously during slow addition of 2N sodium hydroxide (in ethanol) to the neutralization endpoint (pH 8-9). The suspension of the sodium sulfonate derivative was stirred another hour, suction filtered, washed with 95% ethanol on the funnel and subsequently by mechanical slurry in 2000 mi 95% ethanol. The solids were air dried at room temperature to remove most of the solvent before final drying in a forced air oven at 60°C to near constant weight. The yield of the sodium sulfonate derivative, D.S. 1.8, of Udel@ Polysulfone was 1041 grams.

### Example 3

### Udel@ Polysulfone Zinc Sulfonate, D.S. 1.8

A stirred suspension of 133.0 g Udel@ Polysulfone sodium sulfonate derivative, D.S. 1.8, prepared as in Example 2, in 1200 ml water was heated to dissolve the polymer, cooled to room temperature, and the solution centrifuged to remove about 4%. of highly insoluble solids. An aliquot of the total centrifugate containing about 26.3 g sodium sulfonate solids was diluted to about 500 ml with water. A solution of 10.5 g zinc chloride in 20 ml water was added and the resultant hazy solution, pH 6.2, dialyzed in a membrane tube (6000-8000 molecular weight cut-off) surrounded with distilled water for two days. Removal of the water from the dialyzed polymer solution, pH 6.5, by freeze drying gave 25.4 g of purified Udel@ Polysulfone zinc sulfonate, D.S. 1.8, as fluffy white solids.

In an alternate procedure, the solution of the sodium sulfonate derivative in water is prepared and centrifuged after addition of the zinc chloride to give a clarified solution of the zinc sulfonate derivative. Further purification is effected, particularly on a larger scale, by continuous dialysis through a Tri-Ex-1 Hollow Fiber Dialyzer (Extracorporeal Medical Specialties, Inc.) using two passes through the dialyzer at a polymer solution flow rate of 100-200 ml/minute and countercurrent distilled water flow rate of about 500-600 ml/minute. The dialyzed polymer solution is freeze dried to afford the purified polymeric zinc salt.

The polymeric zinc salt was quite hygroscopic and absorbed considerable water (35―40% weight gain) when exposed to relative humidities of both 42 and 75% for about 24 hours. To stabilize the water content of a bulk supply of polymer for clinical studies, the zinc sulfonate obtained via freeze drying was deliberately exposed to laboratory ambient conditions (at a temperature in the range of about 20-25°C and relative humidities that ranged between about 40 and 70%) to allow for maximum moisture uptake for several days to substantially constant weight. Using this procedure, an 8 kg lot of zinc salt was equilibrated to a water content of 21.5% by weight, as determined by thermogravimetric analysis.

The assay values for this lot of polymeric zinc salt are shown in Table 2 and, when corrected for the water content, agreed very well with the theoretical values for the anhydrous zinc salt having a D.S. of 1.8.

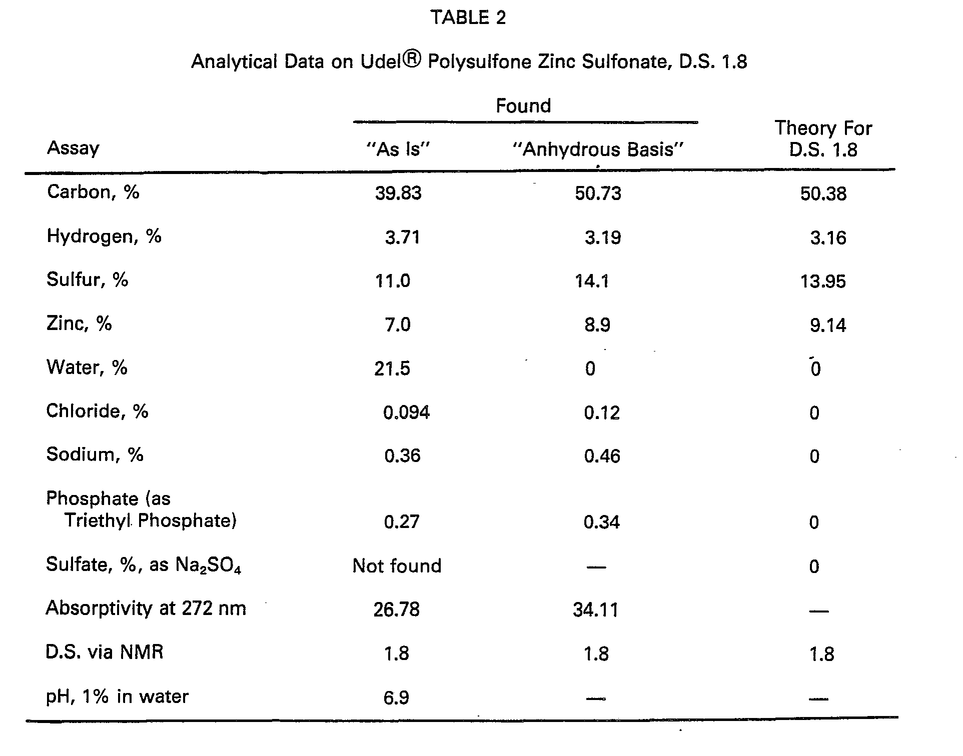

### Example 4

### Udel@ Polysulfone Zinc Sulfonate, D.S. 1.8, Via lon-Exchange of The Sulfonic Acid Derivative

A solution of 5.0 g of the Udel@ Polysulfone sulfonic acid derivative (16.6 milliequivalents of acidity) in 100 ml water was prepared and, after addition of 2.25 g (33.0 meq) zinc chloride, was allowed to stand at room temperature overnight to allow ion-exchange to proceed. The solution was clarified by filtration on an 0.8 pm membrane filter and the filtrate dialyzed in a dialysis membrane tube (M.W. 12,000 cutoff) in water. The hydrochloric acid by-product formed in the ion-exchange reaction was removed to the extent of 94% within one hour of dialysis, as determined by titration of the surrounding water (pH 2.7) with sodium hydroxide. The dialysis was allowed to proceed for several days, and the purified polymer solution stripped free of water under reduced pressure to give 3.7 g of the hygroscopic zinc sulfonate derivative of Udel@ Polysulfone. Analysis: Zinc, 7.08%; Sodium, 0.026%; Absorptivity at 274 nm, 31.9; sulfate and triethyl phosphate were not detectable.

### Example ⁵

### Udel® Polysulfone Sodium Sulfonate, D.S. 0.7

Solutions of 2.2 g (5.0 m moles) polysulfone in 20 ml dry methylene chloride and 0.33 mi·(0.58 g, 4.98 m moles) chlorosulfonic acid in 20 ml methylene chloride were added simultaneously, with stirring, to 40 ml methylene chloride in the reaction flask. The addition time was 21 minutes and the temperature remained constant at 24°C. The pink solution, containing some gummy deposits, was stirred for another hour and was diluted with 100 ml diethyl ether. The clear solution phase was decanted from the gum. On trituration with additional ether, the gum converted to white solids which were collected, washed with ether, and dried to afford 2.5 g of polysulfone sulfonic acid. The D.S., determined by titration of a 2.4925 g sample of the sulfonic acid in 1:1 tetrahydrofuran-water to the neutralization endpoint with 6.9 ml 0.495 N sodium hydroxide solution, was 0.7. Solvent stripping of the neutralized solution gave 2.3 g of the sodium sulfonate derivative of the polysulfone.

### Example 6

### Poly (phenylene ether sulfone) Sodium Sulfonate, D.S. 0.6

A solution of 2.32 g (10.0 m moles) poly (p-phenylene ether sulfone), type 100 P (ICI), in 40 ml dry methylene chloride was added over 21 minutes, at 24-25°C, to a stirred solution of 1.6 g (20.0 m moles) liquid sulfur trioxide in 80 ml methylene chloride. After an additional reaction period of 20 minutes, the reaction mixture was diluted with 20 ml diethyl ether and the white solids collected. The solids were washed by slurrying in 150 ml methylene chloride, filtered, and washed with methylene chloride and ether. The yield of the sulfonic acid derivative was 2.8 g.

- Neutralization of a 2.511 g sample of the sulfonic acid derivative in methanol-water with 8.0 ml 0.641 N sodium hydroxide solution established that the degree of sulfonation (D.S.) was 0.6. Removal of the solvents from the neutralized solution gave 2.5 g of the sodium sulfonate derivative of the polymer.

### Example 7

### Radel@ Polysulfone Sodium Sulfonate, D.S. 1.3

To a stirred suspension of 4.0 g (0.01 mole) powdered Radel® Polysulfone in 40 ml dry methylene chloride was added, over 12 minutes at 24―26°C, a solution of 2.4 g (0.03 mole) liquid sulfur trioxide dissolved in 20 ml methylene chloride containing 1.37 g (7.5 m mole) triethyl phosphate. The suspension was stirred for another 70 minutes at 24-26°C and allowed to stand at room temperature for three days. The solids were filtered, washed with methylene chloride and diethyl ether, and dried to give 3.6 g of the sulfonic acid derivative of the polymer.

The sodium salt was prepared by neutralization of a stirred suspension of 3.5507 g of the sulfonic acid . derivative in 10 ml methanol with standardized sodium hydroxide solution. The solids suspension of the sodium salt was recovered by solvent stripping, dissolution of the residue in 1:1 tetrahydrofuran-water, filtration from some solids (0.5 g) believed to be sodium sulfate, and solvent stripping of the resultant filtrate to fine, tan colored solids weighing 2.3 g; The D.S., corrected for the amount of free sulfuric acid in the sulfonic acid polymer which was recovered as solid sulfate, was 1.3. The D.S. determined by NMR analysis was 1.6.

The plaque barrier oral compositions of this invention may comprise any conventional pharmaceutically acceptable oral hygiene formulation that contains (and is compatible with) an effective amount of a plaque barrier agent as defined herein. Such formulations include, for example, mouthwashes, rinses, irrigating solutions, nonabrasive gel dentifrices, denture cleansers, coated dental floss and interdental stimulator coatings, chewing gums, lozenges, breath fresheners, foams and sprays.

The plaque barrier agents may be present in these formulations in effective concentrations generally in the range of from 0.05 weight percent to as much as 30 weight percent or the limit of compatibility with the vehicle. However, no advantage will be derived from concentrations in excess of 20 weight percent. A preferred concentration range for the plaque barrier agents-in the formulations of the invention is from 0.5 to 10 weight percent. A more preferred range is from 2 to 8 percent by weight, 5% being the presently most preferred concentration in a nonabrasive gel vehicle.

The pH of these plaque barrier preparations should be between pH 5.0 and 10.0, preferably between pH 5.0 and 8.0, more preferably between pH 6.0 and 7.5. Lower pH than 5.0 is undesirable because of the possible enhancement of enamel demineralization.

Suitable conventional pharmaceutically acceptable vehicles that can be employed with the plaque barrier agents to prepare the barrier compositions of this invention may comprise water, ethanol; such humectants as polypropylene glycol, glycerol and sorbitol; such gelling agents as cellulose derivatives, for example, Methocel@, carboxymethylcellulose (CMC TMF) and Klucel@ HF, polyoxypropylene/polyoxyethylene block copolymers, for example, Pluronic@ F-127, Pluronic@ F-108, Pluronic@ P-103, Pluronic@ P-104, Pluronic@ P-105, and Pluronic@ P-123, colloidial magnesium aluminosilicate complexes such as Veegum@, and mucoprotein thickening agents such as Carbopol@ 934; gel stabilizers such as the silicon dioxides, for example, Cab-O-Sil® M5 and polyvinylpyrrolidone; sweeteners such as sodium saccharin; preservatives such as citric acid, sodium benzoate, cetylpyridinium chloride, potassium sorbate, methyl and ethyl parabens; detergents such as sodium lauryl sulfate, sodium cocomonoglyceride sulfonate, sodium lauryl sarcosinate and polyoxyethylene isohexadecyl ether (Arlasolve@ 200) and approved colors and flavors.

The following specific examples will serve further to illustrate the plaque barrier compositions of this invention.

### Example A

### Mouthwash Solution

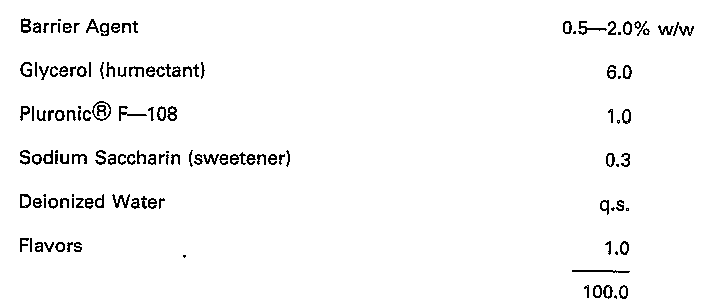

### Example B

### Mouthwash Solution

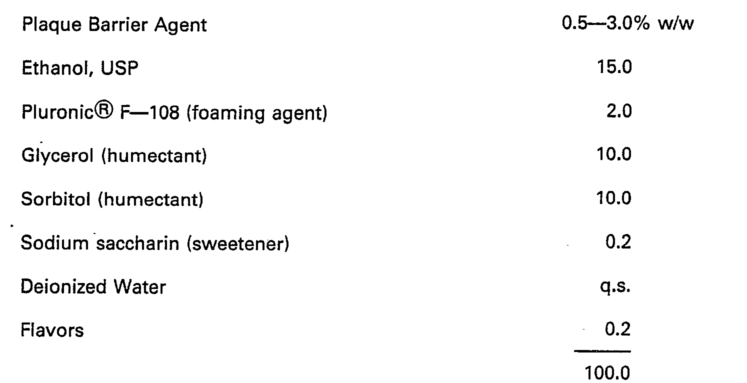

### Example C

### Abrasive Dentifrice Gel

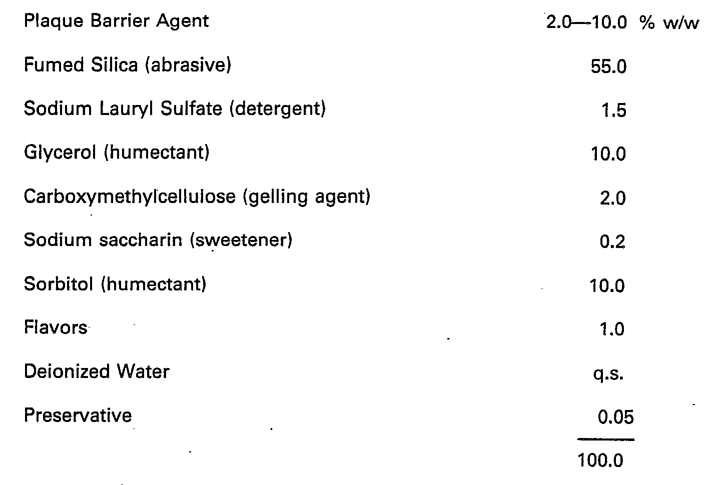

### Example D

### Chewing Gum

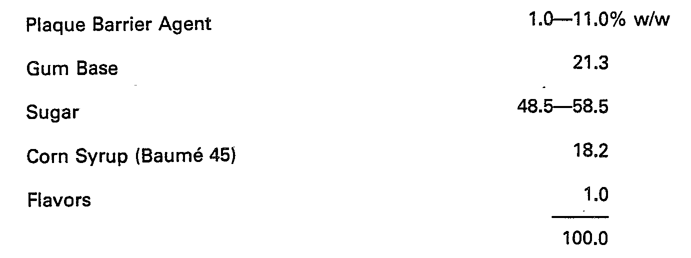

### Example E

### Nonabrasive Gel Dentifrice

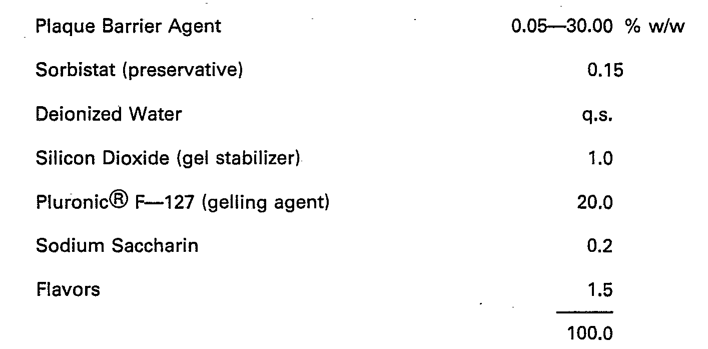

### Example F

The following formulation illustrates a presently preferred nonabrasive gel composition containing a barrier agent in accordance with the present invention.

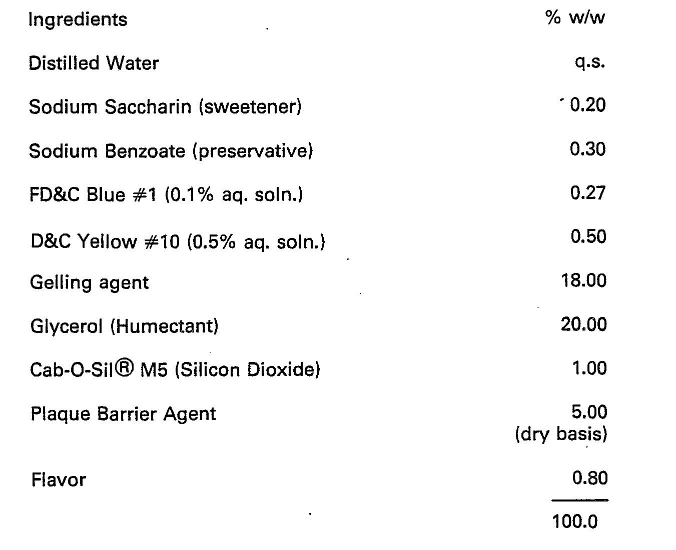

While the details of preparing all of the above formulations are well within the skill of the art, a suggested procedure for preparing the gel formulation of this example will be described for completeness.

In a first container the water, sodium saccharin, sodium benzoate and dyes are mixed. Then the container is put into an ice bath. When the temperature reaches 6°C, the gelling agent is added and the contents mixed slowly until the gelling agent is dissolved. Then the solution is heated to 70°C.

Into a second container is added the glycerin. Then the Cab-O-Sil® M5 is sprinkled in with mixing. Then the plaque barrier agent is added and mixing continued to a smooth paste. The paste is then heated in a water bath with mixing to a temperature of 70°C.

The contents of the first container are added to the second container and blended together until the batch is homogenous while maintianing a 70°C temperature. Then the flavoring is added, all mixing is stopped, and the formulation allowed to settle for approximately one hour. If necessary to remove air bubbles, overnight refrigeration may be employed.

While any pharmaceutically acceptable gelling agent that is compatible with the plaque barrier agent may be employed, a presently preferred gelling agent is Pluronic@ F-127.

These compositions are preferably employed from one to three times daily in a routine oral hygiene program to prevent the attachment of plaque to the teeth.
@ indicates a registered Trade Mark.

## Claims

1. An oral hygiene composition comprising an effective amount for preventing attachment of dental plaque to teeth of a sulfonated poly(arylene ether sulfone) polymer having repeating units selected from the group consisting of structure (A), 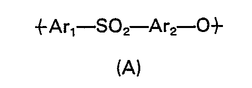 and structure (B), 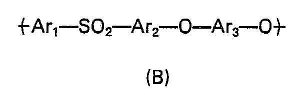 wherein Ar, and Ar₂ are each selected from 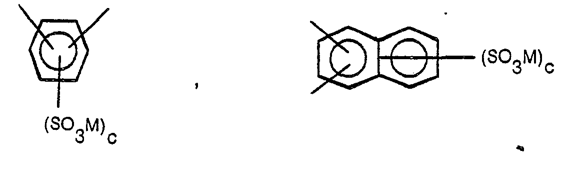 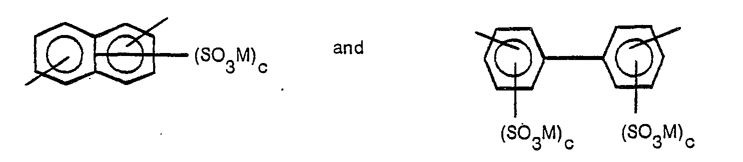 provided further that Ar₂ also can comprise one or more spacing units selected from ―Ar₄―SO₂―Ar₄― and Ar4―SO₂―Ar₄―SO₂―Ar₄― , each Ar₄ in said spacing units being separately selected from 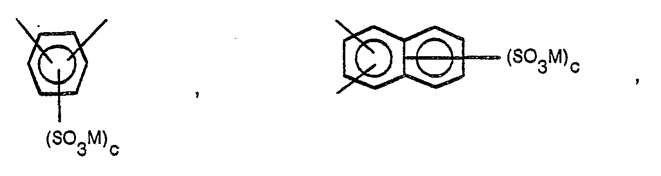 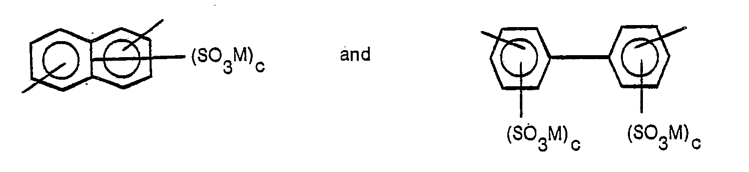 Ar₃ is selected from Ar4 and 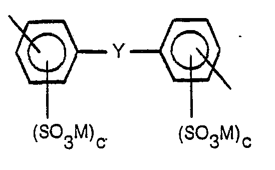 wherein Y is selected from lower alkylene having 1-5 carbon atoms, lower alkylidene having 2-5 carbon atoms, 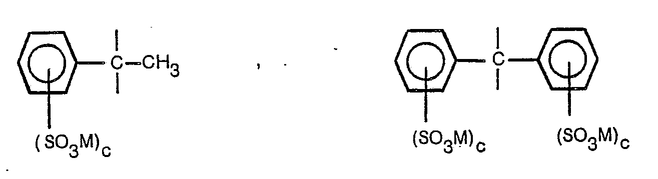 0, S, and S0₂; subscript c being an integer selected from 0, 1, and 2, the average quotient obtained by dividing the sum of thee's within each of repeating units (A) and (B) by the number of aromatic groups in said repeating unit being at least 0.2; and M is selected from lithium, sodium, potassium, calcium, magnesium, zinc, aluminium, hydrogen, ammonium, and substituted ammonium ions derived from pharmaceutically acceptable organic amines, in a pharmaceutically acceptable oral hygiene vehicle compatible with said polymer.

2. The composition of Claim 1 wherein M is a metal selected from potasium, lithium, sodium, calcium, magnesium, zinc and aluminium.

3. The composition of Claim 2 wherein said metal is zinc.

4. The composition of Claim 1, 2 or 3 wherein said quotient is in the range of from 0.2 to 0.5.

5. The composition of Claims 1 to 4 wherein said sulfonated polymer comprises from 0.5 to 10% by weight of the composition, said composition further comprising water, at least 5% by weight of a humectant selected from glycerol and sorbitol, and at least 16% by weight of a gelling agent selected from the block copolymers of polyoxypropylene and polyoxyethylene wherein the polyoxypropylene component has a molecular weight in the range of from 3,000 to 4,000 and the polyoxyethylene comprises between 25 and 80 mole percent of said copolymer.

6. The composition of Claim 5 wherein said sulfonated polymer is present in an amount of from 2 to 8 percent by weight.

7. The composition of claim 5 or 6 wherein the amount of said gelling agent is from 16 percent to 22 percent by weight.

8. The composition of any one of Claims 1 to 7 which further includes an anticaries fluoride compound.

9. The composition of Claim 8 wherein said fluoride compound is sodium fluoride.

10. A composition according to any one of Claims 1 to 9 for use as a plaque prevention agent.

11. A method of preparing an oral hygiene composition comprising mixing an effective amount for preventing attachment of dental plaque to teeth of a sulphonated poly (arylene ether sulphone) polymer as defined in claim 1 with a pharmaceutically acceptable oral hygiene vehicle compatible with said polymer.

## Patentansprüche

1. Eine Zusammensetzung für die Mundhygiene, enthaltend eine zur Verhinderung des Anhaftens von Zahnbelag an Zähnen wirksame Menge eines sulfonierten Poly(arylenethersulfon)polymers mit wiederkehrenden Einheiten, ausgewählt aus der aus die Struktur (A) 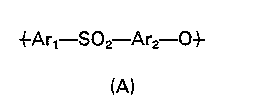 und die Struktur (B) 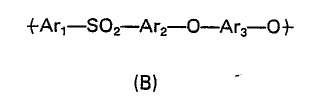 umfassenden Gruppe, worin Ar, und Ar₂ jeweils ausgewählt sind unter 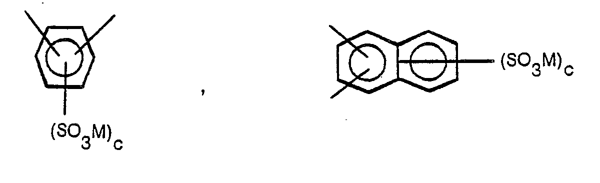 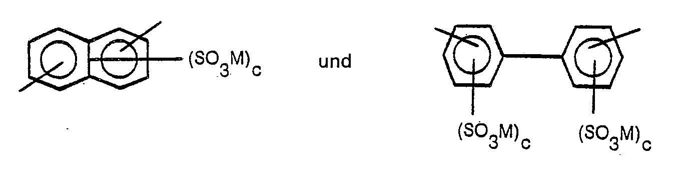 wobei weiterhin Ar₂ eine oder mehrere Abstandseinheiten, ausgewählt unter ―Ar₄―SO₂―Ar₄― und ―Ar₄―SO₂―Ar₄―SO₂Ar₄―; aufweisen kann, wobei jeder Rest Ar₄ in den genannten Abstandseinheiten jeweils für sich ausgewählt sein kann unter 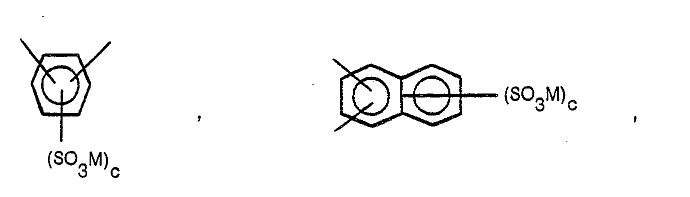 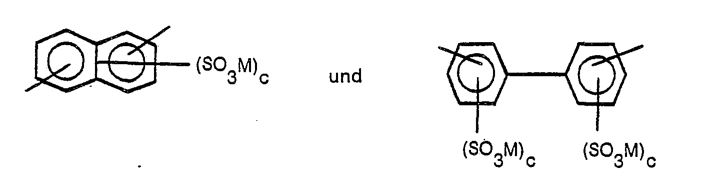 Ar₃ ausgewählt ist unter Ar₄ und 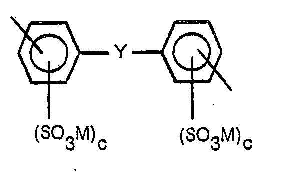 worin Y ausgewählt ist unter Niederalkylen mit 1 bis 5 Kohlenstoffatomen, Niederalkyliden mit 2 bis 5 Kohlenstoffatomen, 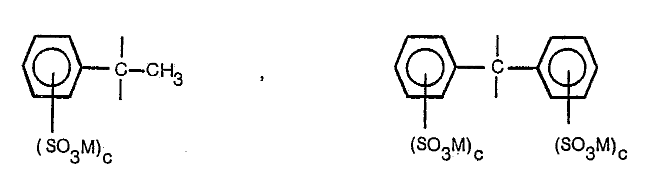 0, S und S0₂; der Index c eine ganze Zahl, ausgewählt unter 0, 1 und 2 bedeutet, wobei der mittlere Quotient, erhalten durch Division der Summe der Indices c innerhalb jeder wiederkehrenden Einheit (A) und (B) durch die Anzahl der aromatischen Gruppen in der genannten wiederkehrenden Einheit, wenigstens 0,2 beträgt; und M ausgewählt ist unter Lithium-, Natrium-, Kalium-, Calcium-, Magnesium-, Zink-, Aluminium-, Wasserstoff-, Ammonium- und substituierten Ammoniumionen, die von pharmazeutisch annehmbaren organischen Aminen abgeleitet sind, in einem mit dem genannten Polymer verträglichen, pharmazeutisch annehmbaren Träger für die Mundhygiene.

2. Die Zusammensetzung nach Anspruch 1, worin M ein unter Kalium, Lithium, Natrium, Calcium, Magnesium, Zink und Aluminium ausgewähltes Metall ist.

3. Die Zusammensetzung nach Anspruch 2, worin das genannte Metall Zink ist.

4. Die Zusammensetzung nach Anspruch 1, 2 oder 3, worin der genannte Quotient in dem Bereich von 0,2 bis 0,5 liegt.

5. Die Zusammensetzung nach den Ansprüchen 1 bis 4, worin das genannte sulfonierte Polymer von 0,5 bis 10 Gew.-% der Zusammensetzung ausmacht, welche Zusammensetzung überdies Wasser, wenigstens 5 Gew.-% eines unter Glycerin und Sorbit ausgewählten Befeuchtungsmittels und wenigstens 16 Gew.-% eines gelbildenden Mittels, ausgewählt unter block copolymeren von Polyoxypropylen und Polyoxyethylen, worin die Polyoxypropylenkomponente ein Molekulargewicht in dem Bereich von 3000 bis 4000 aufweist und das Polyoxyethylen zwischen 25 und 80 Mol-% des genannten Copolymers ausmacht, umfaßt.

6. Die Zusammensetzung nach Anspruch 5, worin das genannte sulfonierte Polymer in einer Menge von 2 bis 8 Gew.-% vorliegt.

7. Die Zusammensetzung nach Anspruch 5 oder 6, worin die Menge des genannten gelbildenden Mittels von 16 bis 22 Gew.-% beträgt.

8. Die Zusammensetzung nach einem der Ansprüche 1 bis 7, welche zusätzlich eine Antikaries-Fluoridverbindung enthält.

9. Die Zusammensetzung nach Anspruch 8, worin die genannte Fluoridverbindung Natriumfluorid ist.

10. Ein Zusammensetzung nach einem der Ansprüche 1 bis 9, zur Anwendung als ein Zahnbelag verhinderndes Mittel.

11. Ein Verfahren zur Herstellung einer Zusammensetzung für die Mundhygiene, umfassend das Vermischen einer zur Verhinderung eines Anhaftens von Zahnbelag an Zähnen wirksamen Menge eines sulfonierten Poly(arylenethersulfon)polymers, wie in Anspruch 1 definiert, mit einem mit diesem Polymer verträglichen, pharmazeutisch annehmbaren Träger für die Mundhygiene.

## Revendications

1. Composition pour l'hygiène orale comprenant une proportion efficace, permettant d'empêcher l'attachement de la plaque dentaire aus dents, d'un polymère sulfoné de poly(arylène-éther-sulfone) ayant des motifs récurrents choisis dans le groupe consistant en structures (A), 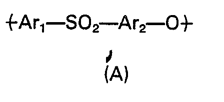 et en structure (B), 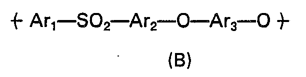 dans lesquelles Ar₁ et Ar₂ sont chacun choisis parmi 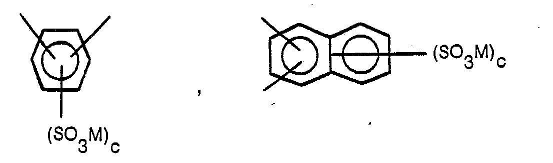 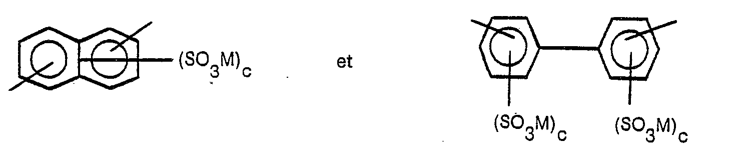 à la condition supplémentaire que Ar₂ puisse comprendre également une ou plusieurs unités d'espacement choisies parmi ―Ar₄―SO₂―Ar₄― et Ar₄―SO₂―Ar₄―SO₂―Ar₄, chaque Ar₄ dans lesdites unités d'espacement étant séparément choisi parmi 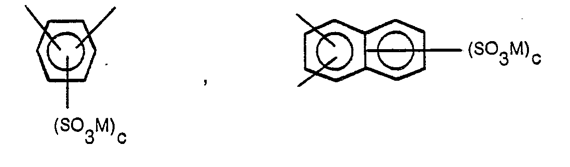 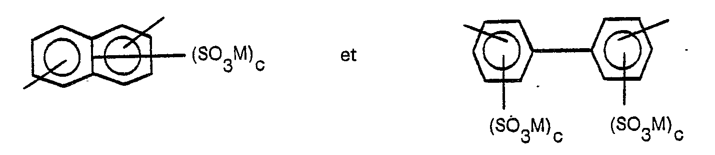 Ar₃ est choisi parmi Ar₄ et 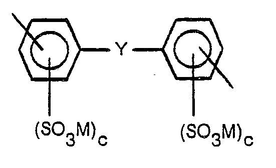 Jans lesquelles Y est choisi parmi les radicaux alkylène inférieurs ayant 1. à 5 atomes de carbone, les radicaux alkylidène inférieurs ayant 2 à 5 atomes de carbone, 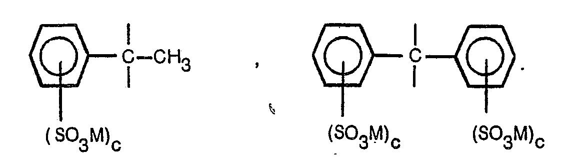 0, S, et SO₂; l'indice c étant un nombre entier choisi parmi 0,.1 et 2, le quotient moyen qu'on obtient en divisant la somme des c dans chacun des motifs récurrents (A) et (B) par le nombre de groupes aromatiques dans ledit motif récurrent étant d'au moins 0,2; et M est choisi parmi le lithium, le sodium, le potassium, le calcium, le magnésium, le zinc, l'aluminium, l'hydrogène, l'ion ammonium, et les ions ammonium substitués provenant d'amines organiques pharmaceutiquement acceptables, dans un véhicule pharmaceutiquement acceptable d'hygiène orale compatible avec ledit polymère.

2. Composition selon la revendication 1, dans laquelle M est un métal choisi parmi le potassium, le lithium, le sodium, le calcium, le magnésium, le zinc et l'aluminium.

3. Composition selon la revendication 2, dans laquelle ledit métal est le zinc.

4. Composition selon la revendication 1, 2 ou 3, dans laquelle ledit quotient est compris entre 0,2 et 0,5.

5. Composition selon les revendications 1 à 4, dans laquelle ledit polymère sulfoné constitue de 0,5 à 10% en poids de la composition, ladite composition comprenant en outre de l'eau, au moins 5% en poids d'un humectant choisi parmi le glycérol et le sorbitol, et au moins 16% en poids d'un agent de gélification choisi parmi les copolymères séquencés de polyoxypropylène et polyoxyéthylène dans lesquels le composant polyoxypropylène présente un poids moléculaire compris entre 3.000 et 4.000 et le polyoxyéthylène constitue de 25 à 80 moles % dudit copolymère.

6. Composition selon la revendication 5, dans laquelle ledit polymère sulfoné est présent à raison de 2 à 8% en poids.

7. Composition selon la revendication 5 ou 6, dans laquelle la quantité dudit agent de gélification est de 16% à 22% en poids.

8. Composition selon l'une quelconque des revendications 1 à 7, qui comprend en outre un composé fluorure anticarie.

9. Composition selon la revendication 8, dans laquelle le composé fluorure est le fluorure de sodium.

10. Composition selon l'une quelconque des revendications l'à à 9, pour utilisation comme un agent de prévention de plaque.

11. Procédé de préparation d'une composition d'hygiène orale, consistant à mélanger une quantité efficace, permettant d'empêcher l'attachement de la plaque dentaire aux dents, d'un polymère sulfoné de poly(arylène-éther-sulfone) tel que défini dans la revendication 1 avec un véhicule d'hygiène orale pharmaceutiquement acceptable compatible avec ledit polymère.
